# EUROPEAN PATENT APPLICATION

(11) **EP 4 790 421 A1**
(43) Date of publication of application: **12.08.2026**
(21) Application number: 26156544.4
(22) Date of filing: 05.02.2026
(51) Int. Cl.: G01N 33/74

(54) **MULTIPLEXED BIOSENSING SYSTEM FOR DETECTING POMC DERIVATIVES**

(30) Priority: 07.02.2025 US 202519048271
(71) Applicant: Zomedica Biotechnologies LLC, Ann Arbor, MI 48108 (US)
(72) Inventor: Harmon, Ian, Golden Valley, MN 55422 (US); Korman, Vicci, Ann Arbor, MI 48108 (US)
(74) Representative: FRKelly

(57) **Abstract**

An analyte detection system and method is described that provides for separate and accurate quantification of the levels of POMC derivatives CLIP and intact ACTH present in a sample. The system may include at least two sensors. In one example of the system of the present invention, primary antibodies are immobilized at the sensor surfaces to capture the analyte (intact ACTH or CLIP), and secondary antibodies are provided to bind to the captured analyte to permit detection of the analyte. The quantification of both intact ACTH and CLIP may provide information about whether a subject (e.g., an animal being evaluated by a veterinarian) is suffering from an ailment. The system may include a cartridge.

## Description

### CROSS-REFERENCE TO RELATED APPLICATIONS

This application claims priority from US patent application No. 19/048,271 filed 7 February 2025.

### TECHNICAL FIELD

The present invention relates generally to analyte detection systems, and more particularly to a biosensor system for measuring ACTH and derivatives thereof in a sample, and a corresponding method for measuring ACTH and derivates thereof in a sample. In one example embodiment, an analyte detection system is configured to capture and detect both intact ACTH and CLIP present in an equine plasma sample. In the aforementioned example embodiment, ACTH and CLIP are captured and measured separately. The system may include a cartridge.

### BACKGROUND AND SUMMARY OF THE INVENTION

Adrenocorticotropic hormone (ACTH) is a peptide involving 39 amino acids. The amino acid sequence for ACTH is Ser-Tyr-Ser-Met-Glu-His-Phe-Arg-Trp-Gly-Lys-Pro-Val-Gly-Lys-Lys-Arg-Arg-Pro-Val-Lys-Val-Tyr-Pro-Asn-Gly-Ala-Glu-Asp-Glu-Ser-Ala-Glu-Ala-Phe-Pro-Leu-Glu-Phe (SYSMEHFRWGKPVGKKRRPVKVYPNGAEDESAEAFPLEF). ACTH is a derivative of Pro-opiomelanocortin (POMC), and is secreted by the anterior pituitary gland of most vertebrates. ACTH stimulates the adrenal glands to produce and release cortisol, which is an important hormone for various bodily functions and/or regulatory processes. Abnormal ACTH levels in a subject (e.g., a mammal such as a horse) may indicate one or more of a variety of health conditions. For example, abnormally high levels of ACTH may indicate overproduction of ACTH due to a pituitary tumor (Cushing's disease), ACTH-producing tumors outside the pituitary gland, and/or primary adrenal insufficiency. As another example, abnormally low levels of ACTH may indicate secondary adrenal insufficiency, steroid use (which may suppress ACTH production), and/or hypopituitarism. Measuring ACTH levels may be useful for detecting such medical conditions.

One method for measuring ACTH levels in a subject involves employing IMMULITE (offered by Siemens Healthineers^{®}). Current equine ACTH diagnostic guidelines are based around IMMULITE. IMMULITE is a group of automated immunoassay analyzers used for lab testing of analyte (e.g., ACTH) levels. The IMMULITE Endocrine Testing System used at the Cornell University Animal Health Diagnostic Center ("Cornell IMMULITE") utilizes IMMULITE immunoassays to measure hormone levels (e.g., ACTH levels) in animals such as horses, cats and dogs. As a specific, non-limiting example, Cornell IMMULITE may be used to detect Equine Cushing's Disease by measuring ACTH levels in a plasma sample taken from a horse. Elevated ACTH levels in the plasma sample may indicate Equine Cushing's Disease. Cornell IMMULITE has been used to establish standards and clinical guidelines in the area.

The results of known ACTH immunoassays are often not fully accurate. For example, the accuracy of known ACTH immunoassays may be affected by the cross reactivity of immunoassay antibodies with a derivative of ACTH known as Corticotropin-Like Intermediate Peptide (CLIP). CLIP is a byproduct of ACTH cleavage, and includes amino acids 18-39 (RPVKVYPNGAEDESAEAFPLEF) of the 39 amino acid sequence SYSMEHFRWGKPVGKKRRPVKVYPNGAEDESAEAFPLEF of ACTH. Assay antibodies intended to bind with intact ACTH may bind with CLIP instead, resulting in said cross reactivity. Higher CLIP levels may result in greater cross reactivity. Known IMMULITE-based techniques for ACTH detection may involve about 20% cross reactivity with CLIP. Seasonal variations may also affect CLIP levels (it is predicted that CLIP levels are highest in horses in the fall), thus cross reactivity issues may vary by season. Without, for example, independent CLIP assessment, the cross reactivity of CLIP with assay antibodies may negatively impact data analysis. With known techniques, a sensor or related assay for independent CLIP assessment is lacking.

Another issue with using known techniques for measuring ACTH levels in animal subject samples is that the known techniques do not yield data indicating and differentiating between both intact ACTH levels and CLIP levels. Although some uncertainty remains regarding the biological significance of CLIP versus full length intact ACTH, it is predicted that each intact ACTH levels and CLIP levels have independent significance in understanding and diagnosing diseases. For example, each intact ACTH levels and CLIP levels may both be relevant in evaluating disease states such as Equine Cushing's Disease (Pituitary pars intermedia disfunction, also referred to as PPID), insulin dysregulation (ID), some combination thereof, or the like. Without being able to independently and accurately quantify both intact ACTH levels and CLIP levels in a single sample, the significance of both intact ACTH levels and CLIP levels may be difficult to further research, evaluate and benefit from.

The aforementioned shortcomings speak to the need for a multiplexed, streamlined system and method for detecting POMC derivatives, wherein both intact ACTH levels and CLIP levels in a sample are separately and accurately quantified.

In view of this, it is beneficial to have a multiplexed biosensing system for intact ACTH and CLIP detection, various exemplary embodiments of which are shown and described in detail herein.

An exemplary multiplexed biosensing system may provide for any number of different accurate testing applications, including, for example, testing at the point of care. Independent CLIP detection data, especially when viewed together with intact ACTH detection data, may provide for more accurate predictive results in the context of determining whether a subject is suffering from a hormonal disease.

According to the present invention in one aspect, an exemplary multiplexed biosensing system for detecting POMC derivatives includes a first and second surface, both configured to receive an amount of a sample. Each surface may be configured to receive sample flow, and may have analyte capture material (e.g., antibodies and/or another analyte capture ligand) thereat for permitting analyte (e.g., intact ACTH or CLIP) in the sample to be captured and detected. Measurement results of the levels of intact ACTH and CLIP found in the sample may be jointly considered to indicate when there is the presence of disease in the animal. The first and second surfaces may define first and second sensors. The first and second sensors may be located at a fluid path. A single volume of sample may be introduced sequentially to the first then second sensor (or vice versa), although such is not required. The first sensor may be configured to detect intact ACTH in the sample. The second sensor may be configured to detect CLIP in the sample. The first and second sensors may be located at a cartridge. The cartridge may be configured to receive the sample. The cartridge may include a port and a fluidic channel configured to transport the sample from the port to at least one of the first and second sensors. The first and second sensors may each be positioned at a sensing portion of the cartridge. The sensing portion of the cartridge may be configured to perform at least one control.

The system may be configured to (e.g., by way of a processor and display) indicate to a user (e.g., a veterinarian) whether the source of the sample (e.g., a horse) is suffering from an ailment (e.g., PPID, ID, or both). An N-term capture antibody may be immobilized at the first sensor for intact ACTH capture. A C-term capture antibody may be immobilized at the second sensor for CLIP capture. A C-term detector antibody may be provided at the first sensor. A C-term detector antibody may be provided at the second sensor. The C-term detector antibodies may include a biotin label. The biotin label may bind to a streptavidin enzyme to attach the enzyme.

The system may include a processor configured to determine total ACTH for the sample based on detected intact ACTH in the sample and detected CLIP in the sample. The processor may be configured to add detected intact ACTH to detected CLIP to determine total ACTH. The processor may be configured to cause total ACTH to be displayed in units of pg/mL. The sample may be an equine plasma sample. The processor may be configured to determine and analyze the ratio of CLIP compared to intact ACTH, and based on the ratio, indicate to a user (e.g., a veterinarian) when the source of the sample (e.g., a horse) is suffering from a disease (e.g., PPID, ID or both).

According to the present invention in another aspect, an exemplary multiplexed biosensing method for detecting POMC derivatives involves providing a first sensor and a second sensor, and configuring each sensor to receive an amount of a sample. The method may comprise positioning each sensor at a fluid path, and causing the amount of sample to be introduced to the sensors sequentially. The method may further comprise configuring the first sensor to detect intact ACTH in the sample. The method may also comprise configuring the second sensor to detect CLIP in the sample. The method may additionally comprise providing a processor configured to determine total ACTH for the sample based on detected intact ACTH in the sample and detected CLIP in the sample. Also, the method may include configuring the processor to add detected intact ACTH to detected CLIP to determine total ACTH. Furthermore, the method may include positioning the first and second sensors at a sensing portion of a cartridge.

An exemplary multiplexed biosensing system and/or method for detecting POMC derivatives may be advantageous for, e.g., accurately detecting and diagnosing diseases in animals such as horses, better evaluating disease states such as PPID and insulin dysregulation, reducing data analysis issues caused by unintended cross-reactivity, better understanding the independent biological significance of each intact ACTH levels and CLIP levels, allowing for earlier diagnosis of various health conditions (e.g., as a result of improved biosensor accuracy and reduced false negatives), allowing for improved health condition assessment guidelines with fewer seasonal changes (e.g., as a result of accounting for CLIP levels which may vary by season), some combination thereof, or the like.

### BRIEF DESCRIPTION OF THE DRAWINGS

Novel features and advantages of the present invention, in addition to those expressly mentioned herein, will become apparent to those skilled in the art from a reading of the following detailed description in conjunction with the accompanying drawings. The present disclosure is illustrated by way of example, and not by way of limitation, in the figures of the accompanying drawings in which like references indicate similar elements. It should be noted that different references to "an" or "one" embodiment in this disclosure are not necessarily to the same embodiment, and such references mean at least one.
**FIG. 1** illustrates exemplary logic for cleavage of Pro-opiomelanocortin (POMC), in accordance with a preferred embodiment of the present invention;
**FIG. 2** illustrates a pair of exemplary sensors for intact ACTH and CLIP detection, in accordance with a preferred embodiment;
**FIG. 3** illustrates exemplary logic for assays at a preferred cartridge of the present invention;
**FIG. 4** illustrates an exemplary multiplexed POMC derivative detection system of the present invention;
**FIG. 5** illustrates a data correlation related to ACTH measurement without CLIP measurement;
**FIG. 6** illustrates a data correlation related to exemplary ACTH measurement with CLIP measurement, in accordance with a preferred embodiment;
**FIG. 7** illustrates data related to ACTH measurement without CLIP measurement;
**FIG. 8** illustrates data related to exemplary ACTH measurement with CLIP measurement, in accordance with a preferred embodiment;
**FIG. 9** illustrates exemplary logic for a preferred multiplexed POMC derivative detection system of the present invention;
**FIG. 10** illustrates a perspective view of an exemplary detector machine and cartridge of the system of FIG. 9; and
**FIG. 11** illustrates a perspective view of an alternative exemplary detector machine, in accordance with a preferred embodiment.

### DETAILED DESCRIPTION OF EXEMPLARY EMBODIMENT(S)

Various embodiments of the present invention will now be described in detail with reference to the accompanying drawings. In the following description, specific details such as detailed configuration and components are merely provided to assist the overall understanding of these embodiments of the present invention. Therefore, it should be apparent to those skilled in the art that various changes and modifications of the embodiments described herein can be made without departing from the scope and spirit of the present invention. In addition, descriptions of well-known functions and constructions are omitted for clarity and conciseness.

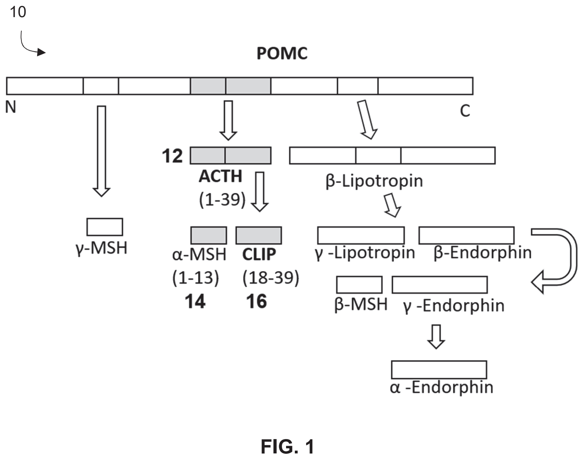

Referring now to FIG. 1, cleavage of Pro-opiomelanocortin (POMC) (10) is shown. POMC is a precursor polypeptide (a long chain molecule comprising amino acids linked together by peptide bonds) having 241 amino acids. POMC is processed inside cells of the pituitary gland, hypothalamus, and other tissues before smaller peptides fragments of POMC (e.g., ACTH, β-Lipotropin) are released into the bloodstream as a result of post-translational cleavage. ACTH (12) is generally produced as a result of POMC being cleaved in corticotroph cells of the pituitary gland by prohormone convertases (particularly, PC1 and PC3). The derivatives of ACTH (12), including α-MSH (14) and CLIP (16), are generally produced as a result of ACTH (12) being cleaved by prohormone convertases (particularly PC2, which may be produced in the pituitary gland and/or other tissues). CLIP (16) includes amino acids 18-39 of the 39 amino acid chain of ACTH (SYSMEHFRWGKPVGKKRRPVKVYPNGAEDESAEAFPLEF), whereas α-MSH (14) includes amino acids 1-13 of the 39 amino acid chain of ACTH. Intact ACTH includes all 39 amino acids of said amino acid chain.

Elevated or decreased levels of ACTH (12) and/or its derivatives may be present in the bloodstream of a subject as a result of disease (e.g., elevated levels as a result of PPID). The levels of ACTH derivatives CLIP and α-MSH, particularly CLIP, may be biologically significant in the context of equine disease states such as PPID and ID. Veterinarians have used assays to detect ACTH (12) presence in samples (blood and/or tissue samples) taken from animal patients. However, known techniques do not quantify CLIP levels in samples, or differentiate between CLIP and intact ACTH levels. Also, with known techniques, CLIP may cause cross reactivity issues with assay antibodies, resulting in less accurate results.

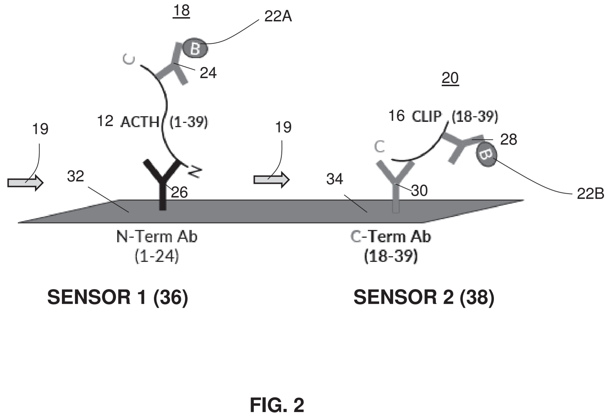

Referring now to **FIG. 2****,** a pair of exemplary sensors 36, 38 are shown. The first sensor 36 may be configured to perform and communicate the results of a first assay 18. The second sensor 38 may be configured to perform and communicate the results of a second assay 20. The first assay 18, which may be performed at a surface 32 of the first sensor 36, may permit the concentration of intact ACTH (12) in a sample (e.g., blood, including fluids derived from blood such as plasma or serum; and/or tissue) introduced to the first sensor 36 to be determined. The second assay 20, which may be performed at a surface 34 of the second sensor 38, may permit the concentration of CLIP (16) in a sample introduced to the second sensor 38 to be determined. The first 36 and second 38 sensors may be separate from and/or isolated from one another. The first 36 sensor may be configured to only detect intact ACTH (12), and not CLIP (16) previously cleaved from ACTH (e.g., to prevent cross reactivity issues). The second sensor 38 may be configured to only detect CLIP (16) previously cleaved from ACTH, and not intact ACTH (12) (e.g., to prevent cross reactivity issues). The sensors 36, 38 may be positioned at a fluid path (illustrated by arrows 19). A volume of sample of the fluid path may first be introduced to the first sensor 36, and thereafter introduced to the second sensor 38 (or vice versa). The sequential introduction of sample to an initial surface (e.g., first sensor 36) having a capture material (e.g., antibody 26) may allow for certain analyte (e.g., intact ACTH) to be removed from the sample volume before it arrives at a subsequent surface (e.g., second sensor 38). This may reduce cross reactivity issues.

The sensors 36, 38 may be located proximate one another. An N-term 1-24 capture antibody (26) may be provided at the sensing surface 32 of the first sensor 36, and the N-term 1-24 capture antibody (26) may be configured to cause intact ACTH (12) from sample introduced to the sensor 36 to bind thereto. A C-term 18-39 detector antibody (24) may be introduced to the first sensor 36, and may be configured to bind to the intact ACTH (12) molecule bound to the primary/capture antibody 26. The C-term antibody (24) may include an antibody label 22A. The antibody label may include biotin (B), which may bind to an enzyme (e.g., biotin molecule binding to streptavidin) to link the enzyme to the antibody 24. The linked enzyme may be configured to react with a substrate to provide signal indicating the concentration of intact ACTH (12) in the sample introduced to the first sensor 36. A C-term 18-39 capture antibody (30) may be provided at the sensing surface 34 of the second sensor 38, and the C-term 18-39 capture antibody (30) may be configured to cause CLIP 16 (cleaved from ACTH) from sample introduced to the sensor 38 to bind thereto. A C-term 18-39 detector antibody (28) may be introduced to the second sensor 38, and may be configured to bind to the CLIP 16 molecule bound to the primary/capture antibody 30. The C-term antibody (28) may include an antibody label 22B. The antibody label may include biotin (B), which may bind to an enzyme (e.g., biotin molecule binding to streptavidin) to link the enzyme to the antibody 28. The linked enzyme may be configured to react with a substrate to provide signal indicating the concentration of CLIP 16 in the sample introduced to the second sensor 38.

By providing for two separate tests 18, 20 for the same sample at two separate sensors 36, 38, both intact ACTH levels and CLIP levels may be determined and communicated to a user (e.g., a veterinarian). An exemplary system employing said sensors 36, 38 may allow the user to better match ACTH values with clinical guidelines. The results of independently measured intact ACTH level and CLIP level may be added together to yield a result that highly satisfies established clinical guidelines (e.g., at least meets IMMULITE industry standards). Exemplary measurement of both intact ACTH levels and CLIP levels may allow for an improved understanding of the involvement of both intact ACTH and CLIP in animal disease states such as, e.g., PPID and ID. For example, an exemplary system may permit a better understanding of biological mechanisms behind variable ACTH and CLIP levels, such as in relation to different disease states, seasonal variations, some combination thereof, or the like. Exemplary independent measurement of both the intact ACTH and CLIP molecules in a sample taken from a subject (e.g., a horse) may allow for earlier diagnosis of disease in the subject, improved guidelines and/or methodology for monitoring, diagnosing and treating subjects, reduced seasonal variability in subject sample data, some combination thereof, or the like.

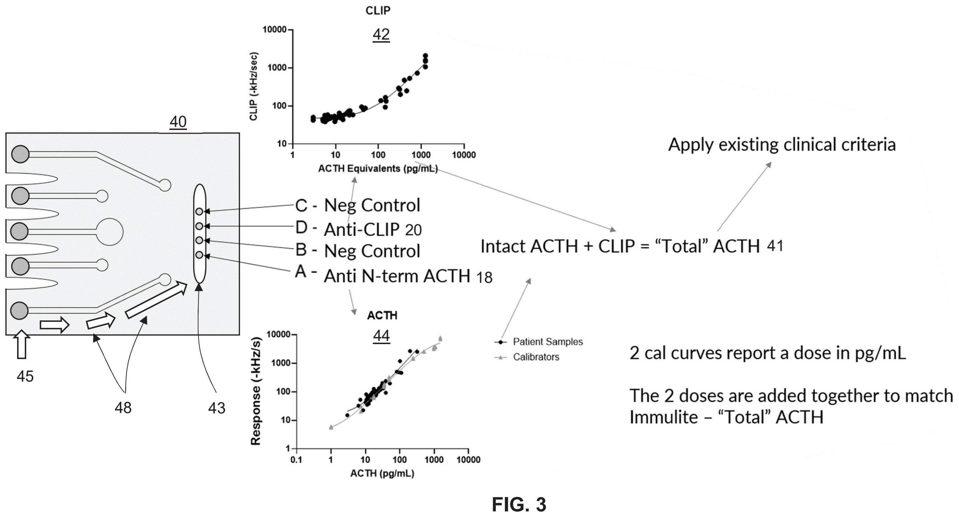

Referring to **FIG. 3****,** exemplary logic for assays at a preferred cartridge 40 is shown. An amount of sample 45 may be introduced to at least one port of the cartridge. The sample 45 may be a horse plasma sample. The cartridge 40 may include one or more fluidic channels 48 configured to transport the sample 45 to a sensing portion 43 of the cartridge 40. The sensing portion 43 of the cartridge may be configured for an anti N-term ACTH (intact ACTH) assay 18 to be performed at a first sensor thereof, and an anti-CLIP assay 20 to be performed at a second sensor thereof. The sensing portion 43 may also be configured for a negative control to be performed thereat with respect to each assay 18, 20. A first negative control may include the primary and secondary antibodies of the anti-CLIP assay 20, and a second negative control may include the primary and secondary antibodies of the anti N-term ACTH assay 18. Each negative control may be configured to receive an amount of control fluid not having any POMC derivatives (as opposed to receiving sample). Each negative control may be configured to provide a baseline assay signal indicative of no intact ACTH or CLIP present.

Signal for each assay may be measured by one or more system readers that may allow for POMC derivative levels to be displayed to a user. Referring to graph 42 of FIG. 3, CLIP level measured from the anti-CLIP assay 20 may be expressed in units of -kHz/sec, and may be expressed over ACTH equivalents (pg/mL). Two calibration curves may report a dose in pg/mL. The two doses may be added together to achieve a "Total" ACTH (41) result (also referred to herein as an "ACTH composite"). The "Total" ACTH result/ACTH composite may satisfy IMMULITE industry standards. Existing clinical criteria may be applied using the ACTH composite. In the graph 44 example of FIG. 3, intact ACTH level measured from the anti N-term ACTH assay is added to CLIP level to yield "Total" ACTH (41), which is expressed in units of pg/mL. A processor may be provided, and configured with instructions to calculate intact ACTH and CLIP levels based on measured assay signal, add intact ACTH level to CLIP level to determine "Total" ACTH level, and cause the aforementioned POMC derivative levels to be displayed at a display screen. The processor may be configured to cause said levels to be displayed in units of pg/mL. The data shown herein is merely illustrative. Any number of different methods for organizing and displaying POMC derivative data including measured intact ACTH and measured CLIP may be employed without departing from the scope of the present invention.

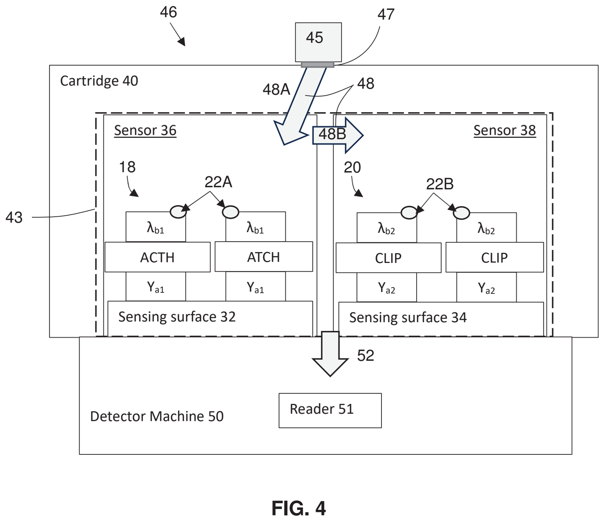

Referring to **FIG. 4****,** an exemplary multiplexed biosensing system 46 for detecting POMC derivatives may include a cartridge 40 having a port 47 for receiving sample 45, a fluidic channel 48, and sensing portion 43. The system 46 may further include a detector machine 50 in communication 52 with the cartridge 40. The sensing portion 43 may include a first sensor 36 and a second sensor 38. A first section 48A of the fluidic channel 48 may be configured to deliver an amount of the sample 45 to the first sensor 36. A second section 48B of the fluidic channel 48 may be configured to deliver the amount of the sample 45 to the second sensor 38. An exemplary sensing portion is not limited to any particular type and/or number of sensors. Although one fluidic channel 48 is illustrated in FIG. 4, an exemplary system is not limited to any particular type and/or number of fluid paths/channels. Any number of different fluid paths/channels (e.g., sequentially arranged, arranged in parallel, or some combination thereof) may be provided to cause one or more sample volumes to flow at different sensors without departing from the scope of the present invention.

At the first sensor 36, a first assay 18 may be performed at a sensing surface 32 of the sensor 36. At the second sensor 38, a second assay 20 may be formed at a sensing surface 34 of the sensor 38. The sensing surfaces 32, 34 may comprise one or more wells (e.g., polystyrene wells) configured to receive primary antibody, sample (which may be diluted), secondary antibody, binding solution (e.g., bovine serum albumin), reagent, substrate, buffer solution (e.g., phosphate buffered saline), detergents (e.g., Tween-20), some combination thereof, or the like. Primary/capture antibody Yₐ₁ may be immobilized at the first sensing surface 32 (e.g., using a binding solution). A buffer solution may be applied thereafter to wash away unbound primary antibody. Thereafter, an amount of sample 45 (e.g., horse plasma, which may be diluted) (various different dilutions may be prepared for multiple sensing surfaces in other embodiments) may be introduced to the sensing surface 32. Analyte other than intact ACTH may be removed or blocked from the sample to address cross-reactivity concerns, although such is not required. Intact ACTH in the sample 45 may bind to the capture antibodies Yₐ₁ at the sensing surface 32. A buffer solution may then be applied to wash away unbound analyte. Thereafter, a secondary/detector antibody λ_{b1} may be introduced to the sensor 36, and the detector antibody λ_{b1} may bind to the intact ACTH bound to the primary antibody Yₐ₁. A buffer solution may then be applied to wash away unbound secondary antibody λ_{b1}.

The secondary antibody λ_{b1} may include a linked enzyme 22A (e.g., biotin, alkaline phosphatase, or the like) configured to react with a substrate (e.g., a chemiluminescent substrate, such as a dioxetane phosphate derivative). The substrate may be provided to react with the linked enzyme 22A to provide a signal of a particular magnitude. The detector machine 50 may include a reader 51 (e.g., an optical reader such as a chemiluminescent reader configured to detect light emission from an alkaline phosphatase reaction) configured to measure the signal magnitude. A system processor may correspond the measured signal magnitude to an intact ACTH concentration (e.g., in pg/mL) for the sample 45.

Primary/capture antibody Yₐ₂ may be immobilized at the second sensing surface 34 (e.g., using a binding solution). A buffer solution may be applied thereafter to wash away unbound primary antibody. Thereafter, an amount of sample 45 (e.g., horse plasma, which may be diluted) (various different dilutions may be prepared for multiple sensing surfaces in other embodiments) may be introduced to the sensing surface 34. Analyte other than CLIP may be removed or blocked from the sample to address cross-reactivity concerns, although such is not required. CLIP in the sample 45 may bind to the capture antibodies Yₐ₂ at the sensing surface 34. A buffer solution may then be applied to wash away unbound analyte. Thereafter, a secondary/detector antibody λ_{b2} may be introduced to the sensor 38, and the detector antibody λ_{b2} may bind to the CLIP bound to the primary antibody Yₐ₂. A buffer solution may then be applied to wash away unbound secondary antibody λ_{b2}. The secondary antibody λ_{b2} may include a linked enzyme 22B (e.g., biotin, alkaline phosphatase, or the like) configured to react with a substrate (e.g., a chemiluminescent substrate, such as a dioxetane phosphate derivative). The substrate may be provided to react with the linked enzyme 22B to provide a signal of a particular magnitude, which may be read by a detector machine 50 reader 51 to determine CLIP concentration (e.g., in pg/mL) in the sample 45. The presence above (or below) a certain threshold of intact ACTH, CLIP, and/or Total ACTH may be indicative of health conditions, such as PPID or ID.

The cartridge 40, detector machine 50, or some combination thereof may be configured with one or more fluid pathways, pumps, controllers, ports, valves, some combination thereof, or the like for introducing the antibodies, binding solution, buffer solution, sample, substrate, and the like to the sensing surfaces 32, 34, and for removing solution as necessary from the sensing surfaces 32, 34. The present invention is not limited to chemiluminescent readers. The present invention may additionally or alternatively employ another optical biosensor reader (for optical signal assays such as, e.g., lateral flow or ELISA) and/or a non-optical biosensor reader such as an electrochemical reader (for electrochemical assays), piezoelectric reader (for piezoelectric assays), or the like. For example, one or more bulk acoustic wave (BAW) resonators may be employed in a fluid path of an exemplary cartridge for piezoelectric analyte detection. The assays described herein are merely illustrative. Any number of different assays involving independent capture and detection of intact ACTH and CLIP may be performed without departing from the scope of the present invention. An exemplary cartridge may be configured for any number of different assay formats.

The cartridge 40 may include various features for interfacing with the detector machine 50 (e.g., valves, pumps, or other fluidic interfaces for pneumatic or liquid-based fluid transfer systems on the detector machine 50). The detector machine 50 may be automated. The detector machine 50 may include any number of different interfaces configured to automatically interact with ports of the cartridge 40. For example, the fluid ports may receive binding solution, bioreceptors (e.g., the primary and secondary antibodies shown in FIGS. 2 and 4), buffer solution, sample, substrate, and the like from fluid flow pathways of the detector machine 50.

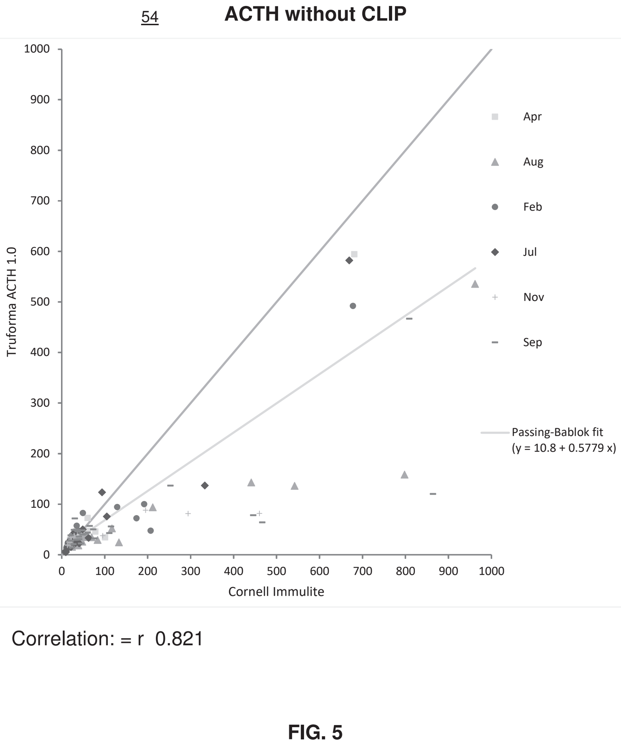

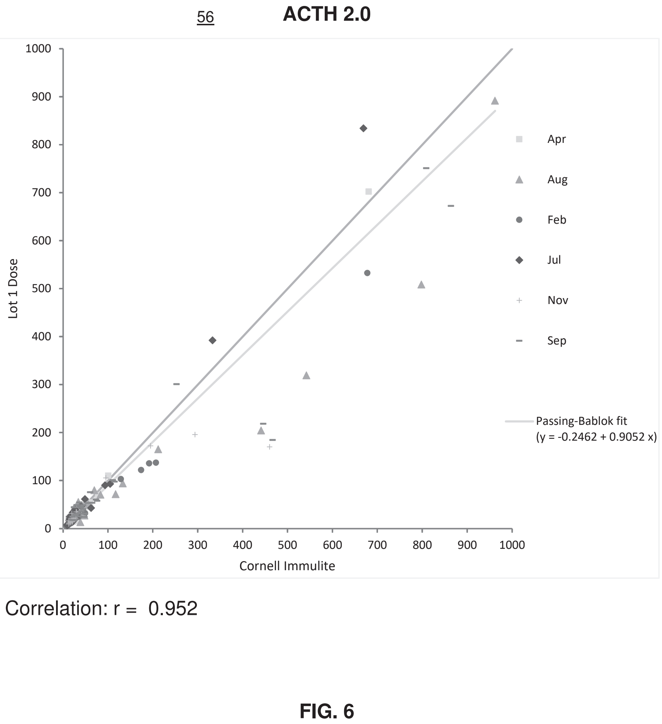

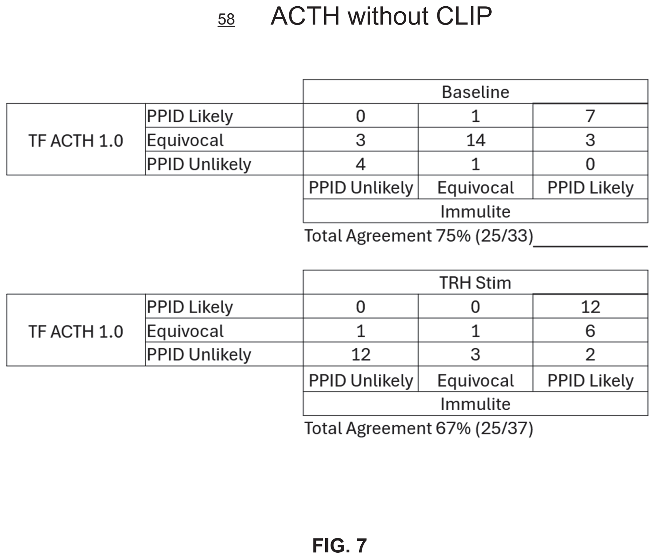

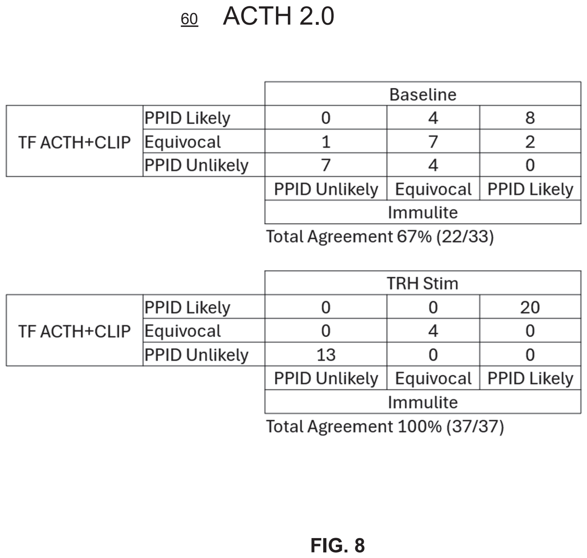

Referring now to **FIGS. 5-8****,** data demonstrating advantages of an exemplary embodiment is shown. Referring specifically to **FIGS. 5-6****,** results of traditional ACTH assays are compared to the Cornell IMMULITE standard in the FIG. 5 graph 54, and results of exemplary ACTH assays of the present invention are compared to the Cornell IMMULITE standard in the FIG. 6 graph 56. Graph 56 demonstrates a much stronger correlation compared to graph 54, thus demonstrating the benefit of an exemplary system incorporating CLIP measurement compared to traditional techniques. Referring now to **FIGS. 7-8****,** results of traditional ACTH assays are compared to the Cornell IMMULITE standard in the FIG. 7 data tables 58, and results of exemplary ACTH assays of the present invention are compared to the Cornell IMMULITE standard in the FIG. 8 data tables 60. The TRH Stim (Thyrotropin-Releasing Hormone Stimulation Test) table of FIG. 8 demonstrates 100% total agreement with the Cornell IMMULITE standard, thus demonstrating that incorporating independent CLIP measurement using an exemplary system greatly improves the results with TRH Stim samples.

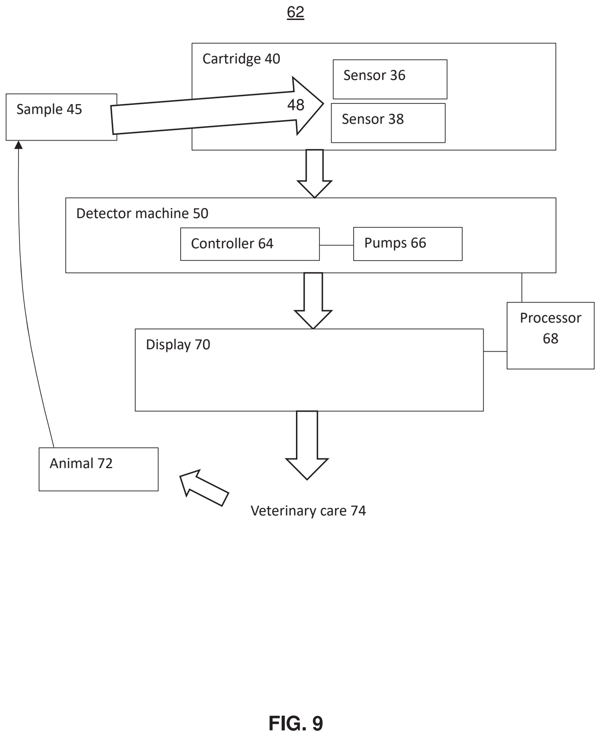

Referring now to **FIG. 9****,** exemplary logic for a preferred multiplexed POMC derivative detection system 62 and method is shown. The system 62 may include a cartridge 40 configured to be loaded into a detector machine 50. At least two sensors 36, 38 may be located at the cartridge 40. A fluidic channel 48 may be configured to transport sample 45 to the sensors 36, 38. The sample 45 may be obtained from an animal 72 patient being cared for by a veterinarian 74. An assay may be performed at each sensor 36, 38. Each assay may involve a negative control. The detector machine 50 may include, e.g., a controller 64 and pumps 66 for regulating assay activity at the cartridge 40. The machine 50 may be configured to read one or more results of the assays at the sensors 36, 38, and a processor 68 linked to the detector machine 50 may be configured to determine analyte concentration based on assay signal. The processor 68 may cause the assay results to be displayed at a digital display 70 of the system 62. The veterinarian 74 may diagnose and treat the animal 72 based on the results. The processor 68 may be configured to determine and analyze the ratio of CLIP compared to intact ACTH, and based on the ratio, indicate to a user (e.g., veterinarian 74) when the source 72 of the sample (e.g., a horse) is suffering from a disease (e.g., PPID, ID or both).

As a specific, non-limiting example, the veterinarian 74 may obtain (e.g., by way of venipuncture) the sample 45 (e.g., a plasma sample) from a horse. A portion (e.g., a droplet obtained using a pipette) of the sample 45 may be introduced to a sample port (not shown) of the cartridge 40 and delivered by way of the at least one fluid pathway 48 to the sensors 36, 38 of the cartridge 40. The processor 68 may regulate various aspects of the system 62, such as, for example, digital display of data at the display 70, operation of the controller 64 and pumps 66 of the detector machine 50, some combination thereof, or the like. Although not required, each assay may be performed multiple times to confirm accuracy and precision of system 62 results.

Aspects of the system 62 may be communicated and/or displayed to system users and/or administrators by way of any number of different computer readable mediums. Aspects of the system 62 may be implemented according to one or more software modules of the processor 68. Software instructions of the system 62 may be executed by the processor 68. System 62 software may be implemented using MATLAB, JAVA, CGI script, Python, some combination thereof, or the like. System 62 software may be stored on an electronic storage medium, and may be executed with the cooperation of a controller and memory. The display 70 may be an electronic display positioned at the façade of the detector machine 50. The present invention is not limited to any particular computing and/or display device, nor is it limited to any particular shape, size, component arrangement and/or design.

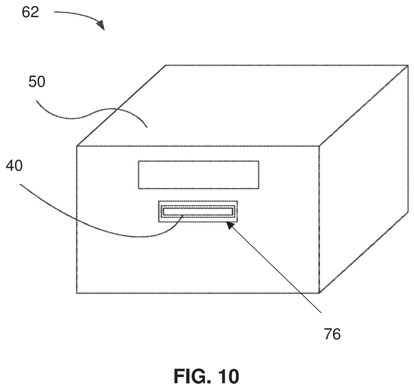

Referring now to **FIGS. 10****,** the detector machine 50 of the system 62 may be configured to receive the cartridge 40 in a receptacle 76 of the detector machine 50. Components of the machine 50, cartridge 40, or some combination thereof may be configured to perform various assay steps (e.g., reagent administration, other solution administration). Additionally, or alternatively, various assay steps may be performed outside of the machine 50. The machine 50 may cause a transducer to measure assay signal for data from the sensors to be obtained. The cartridge 40 or parts of the cartridge 40 may be reusable, recyclable, or disposable. The cartridge 40 may be offered as a portable dry cartridge, meaning that no liquid reagents are stored on the cartridge 40, increasing the storage life of the cartridge 40 and making the cartridge 40 more cost-effective to manufacture.

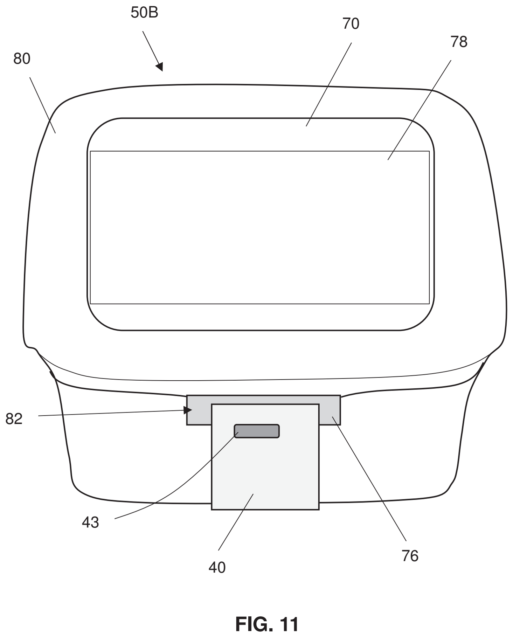

Referring to **FIG. 11****,** an exemplary machine 50B for POMC derivative detection is shown having a housing 80, electronic display screen 70 positioned at an upper portion of the machine 50B, and a cartridge receptacle 76 positioned at a lower portion of the machine 50B. The display screen 70 may be slanted. The cartridge receptacle 76 and cartridge 40 (having sensing portion 43) may each be substantially rectangular, although such is not required. The cartridge receptacle 76 may permit the cartridge 40 to be loaded and transported into an interior portion 82 of the machine 50B, where the machine 50B may cause assay steps to be performed. A reader in the machine 50B may measure assay results and communicate the results to the display screen 70. An interface 78 at the display screen 70 may allow the user to view data, input data, export data, some combination thereof, or the like.

The cartridge 40 may be constructed to receive a liquid sample, to store the sample at least temporarily, to provide sample handling and conditioning, and/or to transfer and meter the sample to a sensor for analysis of one more parameters of the sample. The machine 50B may accommodate any number of different types of fluid samples, and is not limited by sample type. An exemplary system is not limited to any particular type and/or number of cartridges and/or detector machines.

Any embodiment of the present invention may include any of the features of the other embodiments of the present invention. The exemplary embodiments herein disclosed are not intended to be exhaustive or to unnecessarily limit the scope of the invention. The exemplary embodiments were chosen and described in order to explain the principles of the present invention so that others skilled in the art may practice the invention. Having shown and described exemplary embodiments of the present invention, those skilled in the art will realize that many variations and modifications may be made to the described invention. Many of those variations and modifications will provide the same result and fall within the spirit of the claimed invention. It is the intention, therefore, to limit the invention only as indicated by the scope of the claims.

Certain operations described herein may be performed by one or more electronic devices. Each electronic device may comprise one or more processors, electronic storage devices, executable software instructions, and the like configured to perform the operations described herein. The electronic devices may be general purpose computers or specialized computing device. The electronic devices may comprise personal computers, smartphone, tablets, databases, servers, or the like. The electronic connections and transmissions described herein may be accomplished by wired or wireless means. The computerized hardware, software, components, systems, steps, methods, and/or processes described herein may serve to improve the speed of the computerized hardware, software, systems, steps, methods, and/or processes described herein.

## Claims

1. An analyte detection system, comprising:
a first surface, configured to receive an amount of a sample taken from an animal;
a second surface, configured to receive an amount of the sample;
wherein the first surface includes a first analyte capture material configured to capture intact ACTH from the sample;
wherein the second surface includes a second analyte capture material configured to capture CLIP from the sample; and
wherein measurement results of the levels of intact ACTH and CLIP found in the sample are jointly considered to indicate when there is the presence of disease in the animal.

2. The system of claim 1, wherein the first and second surfaces are located at a single cartridge.

3. The system of claim 2, wherein the cartridge is configured to receive the sample.

4. The system of claim 1, wherein the sample is at least one selected from the group of blood and tissue, and wherein the system is configured to indicate to a user, based on detected CLIP and intact ACTH when the animal is suffering from a disease.

5. The system of claim 1, wherein an N-term capture antibody is immobilized at the first surface for intact ACTH capture.

6. The system of claim 1, wherein a C-term capture antibody is immobilized at the second surface for CLIP capture.

7. The system of claim 5, wherein a C-term detector antibody having an enzyme linked thereto is linked to the N-term capture antibody.

8. The system of claim 6, wherein a C-term detector antibody having an enzyme linked thereto is linked to the C-term capture antibody.

9. An analyte detection system, comprising:
a processor;
a first sensor, configured to receive an amount of a sample;
a second sensor, configured to receive an amount of the sample;
wherein the first sensor is configured to detect intact ACTH in the sample;
wherein the second sensor is configured to detect CLIP in the sample; and
wherein the processor is configured to determine total ACTH for the sample based on detected intact ACTH in the sample and detected CLIP in the sample.

10. The system of claim 9, wherein the processor is configured to add detected intact ACTH to detected CLIP to determine total ACTH.

11. The system of claim 9, wherein the system is configured to indicate to a user when a source of the sample is suffering from PPID, ID, or both.

12. The system of claim 9, wherein the processor is configured to determine the ratio of CLIP to intact ACTH, and based on the ratio, indicate to a user when a source of the sample is suffering from a disease.

13. A biological testing method, comprising:
providing a first sensor in a cartridge adapted for analyte detection, said first sensor configured to receive an amount of a sample taken from a mammal;
providing a second sensor in the cartridge, said second sensor configured to receive an amount of the sample taken from the mammal;
configuring the first sensor to detect intact ACTH in the sample;
configuring the second sensor to detect CLIP in the sample.

14. The method of claim 13, further comprising providing a processor configured to determine total ACTH for the sample based on detected intact ACTH in the sample and detected CLIP in the sample.

15. The method of claim 13, further comprising configuring the processor to add detected intact ACTH to detected CLIP to determine total ACTH; and, determining from said total ACTH whether disease is present in the mammal.
